# EUROPEAN PATENT SPECIFICATION

(11) **EP 0 922 017 B1**
(45) Date of publication and mention of the grant of the patent: **03.09.2003**
(21) Application number: 97923648.6
(22) Date of filing: 21.04.1997
(51) Int. Cl.: C07C 2/56, C07C 2/58

(54) **FLUID CATALYTIC ALKYLATION PROCESS**
KATALYTISCHER ALKYLIERUNGSPROZESS IN DER FLÜSSIGPHASE
PROCEDE D'ALKYLATION CATALYTIQUE FLUIDISEE

(30) Priority: 31.05.1996 US 656515
(43) Date of publication of application: 16.06.1999
(73) Proprietor: EXXONMOBIL OIL CORPORATION, Fairfax, VA 22037 (US)
(72) Inventor: HUANG, Tracy, Jau-Hua, Lawrenceville, NJ 08648 (US); WALSH, Dennis, Edward, Richboro, PA 18954-1634 (US)
(74) Representative: Kador & Partner
(86) International application number: US9708143
(87) International publication number: WO97045383

(56) References cited:
- EP-A- 0 437 270
- US-A- 4 300 015
- US-A- 4 384 161
- US-A- 5 073 665
- US-A- 5 095 167
- US-A- 5 292 981

## Description

The instant invention relates to a fluid catalytic isoparaffin-olefin alkylation process in which a circulating fluidized-bed reactor is employed and catalyst is continuously regenerated.

As a result of the curtailment in the use of tetraethyl lead as an octane-improving additive for gasoline, not only has the production of unleaded gasoline increased but the octane number specification of all grades of gasoline has increased as well. Isoparaffin-olefin alkylation is a key route to the production of highly branched paraffin octane enhancers which are to be blended into gasoline.

Alkylation involves the addition of an alkyl group to an organic molecule. Thus, an isoparaffin can be reacted with an olefin to provide an isoparaffin of higher molecular weight. Industrially, alkylation often involves the reaction of C₂-C₅ olefins with isobutane in the presence of an acidic catalyst. Alkylates are valuable blending components for the manufacture of premium gasolines due to their high octane ratings.,

In the past, alkylation processes have included the use of hydrofluoric acid or sulfuric acid as catalysts under controlled temperature conditions. Low temperatures are utilized in the sulfuric acid process to minimize the undesirable side reaction of olefin polymerization and the acid strength is generally maintained at 88 to 94% by the continuous addition of fresh acid and the continuous withdrawal of spent acid. The hydrofluoric acid process is less temperature-sensitive and the acid is easily recovered and purified.

The typical types of alkylation currently used to produce high octane gasoline blending component, that is, the hydrofluoric acid and sulfuric acid alkylation processes, have inherent drawbacks including environmental concerns, acid consumption and disposal of corrosive materials. With the increasing demands for octane and the increasing environmental concerns, it has been desirable to develop an alkylation process based on a solid catalyst system. The catalyst of the present invention offers a refiner a more environmentally acceptable alkylation process than the currently used hydrofluoric and sulfuric acid alkylation processes.

Catalysts comprising large pore crystalline metallosilicates, such as faujasites, REX, REY have been found effective in the solid alkylation processes of the instant invention. Such catalysts are also effective in typical FCC operations. Although these catalysts exhibit high selectivity and activity, they deactivate quickly and require frequent regeneration. This invention discloses a catalytic alkylation process which occurs in a fluidized bed. The catalyst is continually regenerated, a feature which can resolve the aging problem of the catalyst. Riser reactor technology is readily available because it is frequently employed in FCC operations. In this invention, this technology has been adapted to isoparaffin-olefin alkylation.

Crystalline metallosilicates, or zeolites, have been widely investigated for use in the catalysis of isoparaffin-olefin alkylation. For example, U.S. Patent No. 3,251,902 describes the use of a fixed bed of ion-exchanged crystalline aluminosilicate having a reduced number of available acid sites for the liquid phase alkylation of C₄-C₂₀ branched-chain paraffins with C₂-C₁₂ olefins. The patent further discloses that the C₄-C₂₀ branched-chain paraffin should be allowed to substantially saturate the crystalline aluminosilicate before the olefin is introduced to the alkylation reactor.

U.S. Patent No. 3,450,644 discloses a method for regenerating a zeolite catalyst used in hydrocarbon conversion processes involving carbonium ion intermediates.

U.S. Patent No. 3,549,557 describes the alkylation of isobutane with C₂-C₃ olefins using certain crystalline aluminosilicate zeolite catalysts in a fixed, moving or fluidized bed system, the olefin being preferably injected at various points in the reactor.

U.S. Patent No. 3,644,565 discloses the alkylation of a paraffin with an olefin in the presence of a catalyst comprising a Group VIII noble metal present on a crystalline aluminosilicate zeolite, the catalyst having been pretreated with hydrogen to promote selectivity.

U.S. Patent No. 3,647,916 describes an isoparaffin-olefin alkylation process featuring the use of an ion-exchanged crystalline aluminosilicate, isoparaffin/olefin mole ratios below 3:1 and regeneration of the catalyst.

U.S. Patent No. 3,655,813 discloses a process for alkylating C₄-C₅ isoparaffins with C₃-C₉ olefins using a crystalline aluminosilicate zeolite catalyst wherein a halide adjuvant is employed in the alkylation reactor. The isoparaffin and olefin are introduced into the alkylation reactor at specified concentrations and catalyst is continuously regenerated outside the alkylation reactor.

U.S. Patent No. 3,893,942 describes an isoparaffin-olefin alkylation process employing, as catalyst, a Group VIII metal-containing zeolite which is periodically hydrogenated with hydrogen in the gas phase to reactivate the catalyst when it has become partially deactivated.

U.S. Patent No. 3,236,671 discloses the use, in alkylation, of crystalline aluminosilicate zeolites having silica to alumina mole ratios above 3 and also discloses the use of various metals exchanged and/or impregnated on such zeolites.

U.S. Patent No. 3,706,814 discloses another zeolite catalyzed isoparaffin-olefin alkylation process and further provides for the addition of C₅+ paraffins such as Udex raffinate or C₅+ olefins to the alkylation reactor feed and the use of specific reactant proportions, halide adjuvants, etc. U.S. Patent No. 3,624,173 discloses the use, in isoparaffin-olefin alkylation, of zeolite catalysts containing gadolinium.

U.S. Patent No. 3,312,615 discloses an alkylation catalyst comprising crystalline aluminosilicate particles having pore openings of between 6 and 15 Angstroms. These catalysts can be based exchanged with rare earth metals. An Alkylation process is also claimed wherein at least one hydrocarbon stream is contacted with an olefinic stream.

U.S. Patent No. 3,541,180 discloses an alkylation process wherein isobutane is alkylated with ethylene or propylene in the presence of an aluminosilicate catalyst which is suspended as a dust in the gases as they undergo reaction.

U.S. Patent No. 3,851,004 discloses a catalytic process for alkylation of isoparaffin with olefin in the presence of a large pore aluminosilicate. The process can be conducted in a fluidized bed.

U.S. Patent No. 3,917,738 describes a process for alkylating an isoparaffin with an olefin using a solid, particulate catalyst capable of adsorbing the olefin. The isoparaffin with an olefin using a solid, particulate catalyst capable of adsorbing the olefin. The isoparaffin and the olefin are admixed to form a reactant stream in contact with catalyst particles at the upstream end of an adsorption zone after which the reactants are passed concurrently with the catalyst so that a controlled amount of olefin is adsorbed onto the catalyst before the combination of reactants and catalyst is introduced into an alkylation zone. This controlled olefin adsorption is said to prevent polymerization of the olefin during alkylation.

U.S. Patent No. 4,377,721 describes an isoparaffin-olefin alkylation process utilizing, as catalyst, ZSM-20, preferably HZSM-20 or rare earth cation-exchanged ZSM-20.

U.S. Patent No. 4,384,161 describes a process of alkylating isoparaffins with olefins to provide alkylate employing as catalyst a large pore zeolite capable of adsorbing 2,2,4-trimethylpentane, e.g., ZSM-4, ZSM-20, ZSM-3, ZSM-18, zeolite Beta, faujasite, mordenite, zeolite Y and the rare earth metal-containing forms thereof, and a Lewis acid such as boron trifluoride, antimony pentafluoride or aluminum trichloride. The use of a large pore zeolite in combination with a Lewis acid in accordance with this patent is reported to greatly increase the activity and selectivity of the zeolite thereby effecting alkylation with high olefin space velocity and low isoparaffin/olefin ratio.

U.S. Patent Nos. 4,992,615; 5,012,033 and 5,073,665 describe an isoparaffin-olefin alkylation process utilizing, as a catalyst, a zeolite designated as MCM-22. U.S. Patent Nos. 5,258,569 and 5,254,792 disclose isoparaffin olefin alkylation processes which utilize MCM-36 and MCM-49 respectively, as catalysts.

U.S. Patent No. 4,008,291 discloses a moving bed alkylation process in which hydrogen is added to a catalyst reactivation zone but is not added to the reaction zone itself in a controlled amount, as taught in the instant invention.

U.S. Patent No. 4,746,762 discloses a process for upgrading light olefins in a fluidized bed reactor to produce hydrocarbons rich in C₅+ liquids by contacting the olefins with a catalyst comprising a medium pore zeolite. A continuous regeneration process is employed. There is no teaching of isoparaffin-olefin alkylation as in the instant invention. Furthermore, the instant invention employs a large pore zeolite, suitable for adsorbing branched materials. There is also no teaching of the reaction occurring in a riser reactor and no teaching of the use of internal recycle, as in the instant invention.

U.S. Patent No. 4,939,314 discloses a process for regenerating at relatively low pressure a catalyst comprising a medium pore zeolite which has been used in a high pressure fluidized bed oligomerization reactor. There is no teaching of a riser reactor or of the use of a large pore zeolite in this patent. This patent is not directed to isoparaffin-olefin alkylation.
US-A-5,292,981 describes an isoparaffin-olefin alkylation process in a horizontal slurry type reactor. EP-A-437270 describes a liquid phase system and reactor for alkylation with a conventional liquid acid catalyst. US-A-5,073,665 describes an alkylation process in a fixed bed catalyst reactor. According to US-A-5,095,167, the isoparaffin and olefin reactants are in a liquid phase in a reactor vessel. The catalyst is contiuously withdrawn from the lower catalyst phase to a regenerator and returned to the catalyst phase. US-A-4,300,015 describes an isoparaffin-olefin alkylation process in the presence of a zeolite catalyst.

This invention relates to an improved process of reacting an isoparaffin with olefin molecules in the presence of a composite catalyst comprising a large pore zeolite to produce a high octane alkylate. The reaction occurs in a fluidized bed reactor with internal reactor effluent recycle and continuous catalyst regeneration.

The fluid catalytic alkylation process of this invention is conducted by alkylating isoparaffin with olefin in vapor phase in a fluidized bed riser reactor with a solid catalyst.
Hydrogen may optionally be employed. The presence of hydrogen improves catalyst stability and alkylate quality. Furthermore, the use of hydrogen facilitates fluidization in the riser reactor. Following the reaction phase, the solid catalyst is separated from the hydrocarbon stream in a cyclone separator. The catalyst is then steam stripped for the removal of hydrocarbons and gas purged for the removal of water. Both steam stripping and gas purging are conducted in fluidized beds. A portion of the gas purged catalyst is returned to the bottom of the riser reactor, and the remainder is sent to a fluidized bed oxidative regenerator where coke is burned off by air. The regenerated catalyst is recirculated back to the bottom of the riser reactor. Part of the reactor effluent hydrocarbon stream is recycled to the bottom of the riser reactor to increase the isobutane/olefin ratio at the reactor inlet. Part of the reactor effluent hydrocarbon stream is sent to a fractionation tower for the removal of C₅+ alkylate. Unreacted isobutane is recycled from the fractionation tower to the feed stream.

The preferred embodiment of the instant invention involves isobutane alkylation with light olefins, i.e. propylene and butene. These reactants are contacted in vapor phase in a fluidized bed reactor with a rare-earth exchanged faujasite catalyst, as described above.

The alkylation of isobutane with light olefins is important in the manufacture of high octane gasoline blending stocks. Alkylate typically comprises 10 to 15% of the gasoline pool. It has high RON and MON, is low in sulfur content, contains no olefins or aromatics, demonstrates excellent stability and is clean burning.

Figure 1 illustrates the alkylation process of this invention if the reaction occurs in the absence of hydrogen. The feed to the riser reactor, cyclone separator and the vessels for steaming, purging and regeneration of catalyst are depicted, as well as a fractionation tower for recovery of the alkylate product.

Figure 2 illustrates the alkylation process of this invention if hydrogen is employed. Hydrogen enters the riser reactor along with the feed, and is recovered for recycle from the vapors exiting the cyclone separator prior to their entry to the fractionation tower.

### Feed

Feedstocks useful in the present alkylation process include at least one isoparaffin and at least one olefin. The isoparaffin reactant used in the present alkylation process has from about 4 to about 8 carbon atoms. Representative examples of such isoparaffins include isobutane, isopentane, 3-methylhexane, 2-methylhexane, 2,3-dimethylbutane and 2,4-dimethylhexane.

The olefin component of the feedstock includes at least one olefin having from 2 to 12 carbon atoms. Representative examples of such olefins include butene-2, isobutylene, butene-1, propylene, ethylene, pentene, hexene, octene, and heptene, merely to name a few. The preferred olefins include the C₄ olefins, for example, butene-1, butene-2, isobutylene, or a mixture of one or more of these C₄ olefins, with butene-2 being the most preferred. Suitable feedstocks for the process of the present invention are described in U.S. Pat. No. 3,862,258 to Huang et al. at column 3, lines 44-56, the disclosure of which is incorporated by reference.

Hydrocarbon streams containing a mixture of paraffins and olefins such as FCC butane/butene stock may also be employed. The isoparaffin/olefin weight ratio in the feed may range from 1:1 to over 1000:1. Although a ratio of over 100:1 is desirable, a ratio of over 500:1 in the reactor is more desirable and a ratio of over 1000:1 is most desirable. A high isoparaffin/olefin ratio may be achieved by recycle of part of the reactor effluent or by back-mixing of the reactor content.

### Catalyst

The feed, discussed above, is contacted with an alkylation catalyst which comprises a large pore zeolite, optionally in the presence of hydrogen. The catalyst further comprises one or more rare-earth elements. The zeolite component is a large-pore zeolite which is capable of adsorbing 2,2,4 trimethylpentane. The pore diameter of the zeolite is larger than 6 Å, preferably larger than 7Å. Large pore zeolites include the faujasites, such as zeolite X, zeolite Y, and USY. Zeolite X is described more fully in U.S. Patent No. 2,882,244. For purposes of this invention, zeolite Y includes zeolite Y in its as synthesized form, as well as its variant forms including framework dealuminated zeolite Y, e.g., ultrastable Y (USY), described in U.S. Patent No. 3,293,192 and LZ-210 described in U.S. Patent No. 4,503,023. Other suitable zeolites include ZSM-3 (described in U.S. Patent No. 3,415,736), ZSM-4 (more fully described in U.S. Patent Nos. 4,021,947 and 4,091,007), ZSM-18 (described in U.S. Patent No. 5,350,570) ZSM-20 (described in U.S. Patent No. 3,972,983), mordenite (described in U.S. Patent Nos. 5,219,547 and 5,211,935), MCM-22 (described in U.S. Patent Nos. 5,073,665 and 5,105,054), MCM-36 (described in U.S. Patent Nos. 5,310,715 and 5,296,428), MCM-49 (described in U.S. Patent No. 5,236,575), MCM-56 (described in U.S. Patent No. 5,362,697), zeolite-L (described in U.S. Patent Nos. 4,908,342 and 5,063,038), zeolite beta (described in U.S. Patent Nos. 5,164,170 and 5,160,169). Faujasites are preferred in the instant invention. The entire disclosures of the patents referred to in this paragraph are expressly incorporated by reference.

The zeolites are preferably partially or fully exchanged with at least one rare-earth cation, such as cations of lanthanum or cerium. Mixtures of rare-earth cations may also be used. Other cations, such as Ca⁺², may also be used. The large pore zeolite can be one in which the sodium content is no more than 1.0% wt.

Prior to its use as an alkylation catalyst in the process of this invention, the catalyst must be at least partially dehydrated. This dehydration can be accomplished by heating the catalyst to a temperature in the range of from 200°C, to 595°C, in an atmosphere such as air, nitrogen, etc., and at atmospheric, subatmospheric or superatmospheric pressures for a period of from between 30 minutes to 48 hours. Dehydration can also be performed at room temperature merely by placing the catalyst in a vacuum but a longer time will be required to achieve a suitable degree of dehydration.

The catalyst can be shaped into a wide variety of particle sizes. Generally speaking, the particles can be provided in the form of a powder. The particles are in the range from 20 to 200 microns, preferably 50 to 150 microns and more preferably 70 to 80 microns. In cases where the catalyst is molded, such as by extrusion, the crystals can be extruded before drying or partially dried and then extruded.

It is desired to incorporate the catalytically active catalyst crystalline material with another material, i.e., a binder, which is resistant to the temperatures and other conditions employed in the isoparaffin alkylation process of this invention. Suitable binder materials include active and inactive materials such as clays, silica and/or metal oxides such as alumina. These can be either naturally occurring or provided in the form of gelatinous precipitates or gels including mixtures of silica and metal oxides. Use of a binder material in conjunction with the catalytically active crystalline material, i.e., combined therewith, which itself is catalytically active may change the conversion and/or selectivity of the catalyst. Inactive materials suitably serve as diluents to control the amount of conversion so that products can be obtained economically and in a controlled fashion without having to employ other means for controlling the rate of reaction. These materials can be incorporated into naturally occurring clays, e.g., bentonite and kaolin, to improve the crush strength of the catalyst under commercial operating conditions. Good crush strength is an advantageous attribute for commercial use since it prevents or delays breaking down of the catalyst into powder-like materials.

Naturally occurring clays which can be composited with the present catalyst crystals include the montmorillonite and kaolin family, which families include the subbentonites, and the kaolins commonly known as Dixie, McNamee, Georgia and Florida clays or others in which the main mineral constituent is halloysite, kaolinite, dickite, nacrite, or anauxite. Such clays can be used in the raw state as originally mined or initially subjected to calcination, acid treatment or chemical modification. Binders useful for compositing with catalyst crystals also include inorganic oxides, notably alumina.

The alumina binder may undergo a phase transformation during calcination, whereby the water solubility of the alumina is decreased. The hydroxyl content of the alumina may be decreased by calcination. In particular, calcination may transform the pseudoboehmite form of alumina into gamma-alumina.

Apart from or in addition to the foregoing binder materials, the present catalyst crystals can be composited with an inorganic oxide matrix such as silica-alumina, silica-magnesia, silica-zirconia, silica-thoria, silica-beryllia, silica-titania as well as ternary compositions such as silica-alumina-thoria, silica-alumina-zirconia, silica-alumina-magnesia, silica-magnesia-zirconia, etc. It may be advantageous to provide at least a part of the foregoing matrix materials in colloidal form so as to facilitate extrusion of the catalyst component(s).

The relative proportions of finely divided catalyst crystals and inorganic oxide matrix can vary widely with the catalyst crystals content ranging from about 1 to 95% by weight and more usually, particularly when the composite is prepared in the form of beads, in the range of 2 to 80 wt.% of the composite.

### OPERATING CONDITIONS

### A. Riser Reactor

The operating temperature of the alkylation process herein can extend over a fairly broad range, e.g., from temperatures lower than 483°C (900°C), preferably lower than 316°C (600°F) and more preferably below 149°C (300°F). The practical upper operating temperature will often be dictated by the need to avoid an undue occurrence of undesirable side reactions.

The pressures employed in the present process are quite low, from atmospheric pressure to 791 kPa (100 psig), and preferably from 274-377 kPa (25 psig to 40 psig). The gas velocity in the riser reactor is at least 61 cm/s (2 ft./sec.), preferably higher than 3,05 m/s (10 ft./sec.), more preferably higher than 6,1 m/s (20 ft./sec). The residence time in the riser reactor is controlled in such a way that complete or near complete olefin conversion is obtained. When hydrogen is employed the mole ratio of hydrogen to olefin in the feed stream can range from 0.1:1 to 1000:1, preferably from 2:1 to 50:1.

The amount of catalyst used in the present alkylation process can be varied over relatively wide limits. The weight ratio of catalyst to hydrocarbon feed can range from 100:1 to 1:100. It will, of course, be realized by those skilled in the art that the amount of catalyst selected for a particular reaction will be determined by several variables including the reactants involved as well as the nature of the catalyst and the operating conditions employed.

The isoparaffin reactant used in the present alkylation process may be one possessing up to 20 carbon atoms and preferably one having from 4 to 8 carbon atoms as, for example, isobutane, 3-methylhexane, 2-methylbutane, 2,3-dimethylbutane and 2,4-dimethylhexane.

The olefin reactant employed herein generally contains from 2 to 12 carbon atoms. Representative examples are ethene, propene, butene-1, butene-2, isobutene, pentenes, hexenes, heptenes and octenes. Particularly preferred are C₃ and C₄ olefins and mixtures thereof.

In general, the mole ratio of total isoparaffin to total olefin alkylating agent in the hydrocarbon feed can be from 1:1 to 100:1 and is preferably in the range of from 5:1 to 50:1. An internal isoparaffin/olefin ratio of above 500 in the reactor is desirable. The internal recycle ratio of the reactor effluent hydrocarbon stream to the combined feedstream (alkylation feed plus externally recycled isobutane) can range from 1:1 to 500:1, preferably from 10:1 to 100:1. The isoparaffin and/or olefin reactants are in the vapor phase in this invention. The reactants can be neat, i.e., free from intentional admixture of dilution with other material, or the reactants can be brought into contact with the catalyst composition with the aid of a carrier gas such as hydrogen.

### B. Regeneration Procedure

### 1. Stripping Zone

The conditions used in stripping the catalyst are preferably as low as is consistent with the cost of compressing stripped gas to be returned to the process. It is generally beneficial to operate the stripper in the range from 274 to 377 kPa (25 to 40 psig).

The preferred stripper operating temperature is in the range from 121° to 482°C (250° to 900°F) and the pressure in the range from 274 to 377 kPa (25 to 40 psig). The operating pressure is relatively constant though it may vary within a narrow range on either side of the desired pressure. When the stripping medium is steam as is generally the case, the conditions of pressure and temperature are dictated by limits of the deactivation curve of the catalyst, above which curve the catalyst is unacceptably deactivated. The most preferred stripping conditions are chosen to provide the highest temperature and lowest economic pressure at which the catalyst will not be affected deleteriously.

### 2. Purging Zone

The gas purger is employed to purge hydrocarbons from the catalyst that were not removed by the stripper. Excess water is also removed from the catalyst in the purging zone.
Suitable conditions include a temperature range from 121°C (250°F) to 482°C (900°F). The pressure range could be from 101 to 791 kPa (0 to 100 psig), preferably 274 to 377 kPa (25 to 40 psig), as in the riser. The gas purger is pressurized with gas before the catalyst is allowed to flow into the purger from the stripper. Typically the pressure is high enough to avoid damaging the valve in the recirculation line due to too high a velocity, though below sonic, of the recirculation catalyst stream. The recirculation line returns some catalyst to the bottom of the riser after purging but prior to regeneration. The purging process is a continuous one.

### 3. Regeneration Zone

The conditions for regenerating coked up catalyst are generally substantially the same irrespective of the mode of operation of the reactor, and again, are dictated by the economics of operating the chosen process embodiment under pressure. The preferred regenerator operating temperature is in the range from 483° to 594°C (900° to 1100°F) and the pressure in the range from 240 to 445 kPa (20 to 50 psig). The regenerator may be operated continuously, though stripped catalyst is flowed to it only intermittently. If the "coke make" and the rate of flow of recirculated catalyst are each small enough, stripped catalyst may be accumulated in the regenerator until there is enough to regenerate it economically. Under appropriate conditions, the regenerator may thus be operated semi-continuously. Because of the process conditions under which the catalyst is stripped of hydrocarbons, and under which it is coked up, it may be activated by air alone, a flue gas recycle being unnecessary to doctor the air and keep the regenerator outside the explosive limits of its contents.

The preferred reaction is isobutane with butenes, in the presence of REUSY catalyst. Preferred conditions are disclosed.

In a preferred process for isobutane alkylation with butenes in the absence of hydrogen with a commercial FCC (fluid catalytic cracking) catalyst containing RE-USY the average particle size of the FCC catalyst is 70 micron. Alkylation feed (line 1) with a isobutane/butene (I/O) mole ratio of 1:1 is mixed with the externally recycled isobutane stream from the fractionation tower to form a combined feed stream which has a increased I/O ratio of 20:1. After preheating, this combined feed stream is further mixed with the internally recycled reactor effluent hydrocarbon stream in a volume ratio of 1:50 to form the reactor feed stream. The I/O ratio of this reactor feed stream is then approximately 1000:1. This feed stream and the regenerated catalyst are fed into the bottom of the fluidized-bed riser reactor which is operated at 274 kPa (25 psig).
Solid and hydrocarbon streams are separated by a cyclone. The separated solid catalyst is transported via line 10 to a steam stripper where hydrocarbons are stripped off at 232°C (450°F) and 274 kPa (25 psig). The resulting stripped catalyst is then sent via line 11 to a gas purger where at least part of the water on the catalyst is removed at 399°C (750°F) and 274 kPa (25 psig). Part of the gas-purged catalyst is returned via lines 13 and 15 to the bottom of the riser reactor, and part of the gas-purged catalyst is sent to the regenerator where "coke" is burned off by air at 538°C (1000°F) and 274 kPa (25 psig). The oxidatively regenerated catalyst is returned to the bottom of the riser reactor. Heat exchanger may be employed to adjust the catalyst temperature. Part of the reactor effluent hydrocarbon stream is recycled to the combined feed stream as stated above. Part of the effluent hydrocarbon stream is sent to the fractionation section where C₅+ alkylate is separated and isobutane is recycled via line 8 to the alkylation feed.

In another preferred process for isobutane alkylation with butenes in the presence of hydrogen with a commercial FCC (fluid catalytic cracking) catalyst containing Re-USY the average particle size of the FCC catalyst is about 70 micron. The process scheme and the operating conditions for the riser reactor, the steam stripper, the gas purger and the regenerator are all the same as those described in Example 1, except the following: Hydrogen is mixed with the feed stream to achieve a mole ratio of hydrogen to olefin of 20:1 at the reactor inlet and the resulting mixture is fed into the riser reactor. A high pressure separator is installed prior to the fractionation tower to separate hydrogen from the hydrocarbon stream. The separated hydrogen is recycled to line 16.

### Examples

### Example 1

A laboratory-prepared fluidized-bed catalyst containing 50% alumina and 50% REX (rare-earth containing faujasite) in the particle size range of 50 to 200 microns was employed in this fluidized-bed catalytic alkyulation experiment. Twenty cc of this solid catalyst was charged to 1.27 cm (0.5") I.D. quartz fluidized-bed reactor. The catalyst was fluidized with a nitrogen stream of 200 cc/min and dried at 250° for 2 hours. The reactor temperature was then reduced to 70°C, and then the nitrogen gas was shut off. Simultaneously, an isobutane/butene-2 feedstock containing 1 wt.% of n-hexane (as an internal standard for analytical purposes) with an isobutane/butene-2 molar ratio of 50:1 was fed into the reactor at a liquid pumping rate of 58.5 ml/hr. After 10 minutes on stream, the reactor effluent was analyzed by gas chromatograph. The conversion of butene-2 was over 99 wt.%.

The C₅+ alkylate showed the following composition:

| COMPONENT | wt.% |
|---|---|
| isopentane | 57.25 |
| 2,3-dimethylbutane | 12.69 |
| 2-methylpentane | 3.63 |
| 3-methylpentane | 2.59 |
| 2,4-dimethylpentane | 4.40 |
| 2,2,3-trimethylbutane | 0.52 |
| 2,3-dimethylpentane | 2.33 |
| 3-methylhexane | 0.52 |
| 2,2,4-trimethylpentane | 4.92 |
| 2,5-dimethylhexane | 1.30 |
| 2,4-dimethylhexane | 0.78 |
| 2,2,3-trimethylpentane | 1.55 |
| 2,3,4-trimethylpentane | 2.85 |
| 2,3,3-trimethylpentane | 3.63 |
| 2,3-dimethylhexane | 1.04 |
| Total | 100 |

The results show that C_{c}+ was formed in the fluidized-bed operation even with a contact time of only 5.5 seconds.

### Example 2

The used catalyst from Example 1 was steam-stripped at 232°C for 30 minutes and then purged with nitrogen (220 cc/min) at the same temperature for 2.5 hours. Subsequently, the catalyst was heated to 538°C in the presence of air at 80 cc/min and the oxidative coke burning was conducted at 538°C for one hour. Then the reactor temperature was reduced to 70°C and the nitrogen flow was stopped. The same isobutane/butene-2 feedstock as used in Example 1 was then fed into the reactor at the same pumping rate of 58.5 ml/hr. After 10 minutes on stream, the reactor effluent was analyzed by gas chromatograph. The conversion to butene-2 was over 99% and the C₅+ alkylate showed the following composition:

| COMPONENT | wt.% |
|---|---|
| isopentane | 16.58 |
| n-pentane | 0.52 |
| 2,3-dimethylbutane | 10.62 |
| 2-methylpentane | 3.63 |
| 3-methylpentane | 4.15 |
| 2,4-dimethylpentane | 4.92 |
| 2,2,3-trimethylbutane | 1.04 |
| 2-methylhexane | 0.52 |
| 2,3-dimethylpentane | 3.63 |
| 3-methylhexane | 0.78 |
| 2,2,4-trimethylpentane | 9.59 |
| 2,5-dimethylhexane | 1.04 |
| 2,4-dimethylhexane | 1.80 |
| 2,2,3-trimethylpentane | 3.37 |
| 2,3,4-trimethylpentane | 13.73 |
| 2,2,3-trimethylpentane | 11.66 |
| 2,3-dimethylhexane | 5.96 |
| 3,4-dimethylhexane | 3.37 |
| C₉+ | 3.10 |
| Total | 100.00 |

The results above show that the oxidatively regenerated REX catalyst not only exhibited the same activity but also improved the selectivity of the isooctanes.

## Claims

1. An isoparaffin-olefin alkylation process that comprises the following steps:
(a) introducing fresh feed reactants comprising isoparafins and olefins into a fluidized bed riser reactor and introducing a particulate, fluidizable catalyst comprising a large pore zeolite into the riser reactor, the zeolite being capable of absorbing 2,2,4-trimethylpentane, and the catalyst particles having a particle size in the range of from 20 to 200 microns;
(b) reacting the isoparaffin and olefin reactants under alkylation conditions in the presence of the catalyst within the riser reactor to form a riser effluent comprising an alkylate product and unreacted isobutane, wherein the isoparaffin and olefin reactants are in the vapor phase within the riser reactor;
(c) separating the catalyst from the riser effluent in a cyclone;
(d) recirculating at least a portion of the riser effluent to the riser reactor;
(e) passing the catalyst to a stripping zone comprising a fluidized bed, and stripping the catalyst with stream within the stripping zone to remove hydrocarbons;
(f) passing the stripped catalyst from the stripping zone to a purging zone comprising a fluidized bed, and contacting the stripped catalyst with a gas for the removal of water and hydrocarbons;
(g) recirculating at least a portion of the purged catalyst to the riser reactor, and passing another portion of the purged catalyst to a regeneration zone comprising a fluidized bed, and oxidatively regenerating the purged catalyst within the regeneration zone to produce a catalyst essentially free of deposited coke; and
(h) recirculating the regenerated catalyst to the riser reactor.

2. The process of claim 1, wherein the riser effluent is passed to a separator for the removal of alkylate product and isobutane, the isobutane being recycled to the riser reactor.

3. The process of claim 1, wherein the alkylated product is a C₅+ alkylate.

4. The process of claim 1, wherein the reaction within the riser occurs in the presence of hydrogen.

5. The process of claim 1, wherein the reaction within the riser occurs in the absence of hydrogen.

6. The process of claim 4, wherein a range of mole ratios of hydrogen to olefin of 0.1:1 to 1000:1 is employed.

7. The process of claim 4, wherein a range of mole ratios of hydrogen to olefin of 2:1 to 50:1 is employed.

8. The process of claim 1, wherein the large pore zeolite is selected from the group consisting of faujasites, zeolite beta, ZSM-3, ZSM-4, ZSM-18, ZSM-20, mordenite, MCM-22, MCM-36, MCM-49, MCM-56 and zeolite L.

9. The process of claim 8, wherein the faujasites are selected from the group consisting of zeolite X, zeolite Y and USY.

10. The process of claim 1, wherein the large pore zeolite is partially or fully exchanged with a cation or cations selected from the rare-earth metals.

11. The process of claim 10, wherein the large pore zeolite is REY or REUSY.

12. The process of claim 1, wherein the large pore zeolite comprises no more than 1.0 wt.% sodium.

13. The process of claim 1, wherein the catalyst comprising the large pore zeolite further comprises a binder.

14. The process of claim 1, wherein alkylation conditions in the riser reactor-include pressures in the range from 101 to 791 kPa (0 to 100 psig) and temperatures below 483°C.

15. The process of claim 1, wherein the catalyst comprises particles which range in size from 20 to 200 microns in size.

## Patentansprüche

1. Verfahren zum Alkylieren von Isoparaffin-Olefin, das die folgenden Schritte aufweist:
(a) Einführen einer frischen Reaktanten-Beschickung, die Isoparaffine und Olefine umfaßt, in einen Steigrohrreaktor mit Wirbelbett und Einführen eines partikelförmigen, verwirbelbaren Katalysators, der einen Zeolith mit großen Poren umfaßt, in den Steigrohrreaktor, wobei der Zeolith 2,2,4-Trimethylpentan absorbieren kann und die Katalysatorpartikel eine Partikelgröße im Bereich von 20 bis 200 µm aufweisen;
(b) Umsetzen der Isoparaffin- und Olefin-Reaktanten bei Alkylierungsbedingungen in Gegenwart des Katalysators im Steigrohrreaktor, wodurch ein Abfluß des Steigrohrs erzeugt wird, der ein Alkylatprodukt und unreagiertes Isobutan umfaßt, wobei die Isoparaffin- und Olefin-Reaktanten im Steigrohrreaktor in der Dampfphase vorliegen;
(c) Abtrennen des Katalysators vom Abfluß des Steigrohrs in einem Zyklon;
(d) Rezirkulieren von zumindest einem Teil des Abflusses des Steigrohrs zum Steigrohrreaktor;
(e) Leiten des Katalysators zu einer Abstreifzone, die ein Wirbelbett umfaßt, und Abstreifen des Katalysators in der Abstreifzone mit Dampf, um Kohlenwasserstoffe zu entfernen;
(f) Leiten des abgestreiften Katalysators aus der Abstreifzone zu einer Spülzone, die ein Wirbelbett umfaßt, und Inkontaktbringen des abgestreiften Katalysators mit einem Gas, um Wasser und Kohlenwasserstoffe zu entfernen;
(g) Rezirkulieren von zumindest einem Teil des gespülten Katalysators zum Steigrohrreaktor und Leiten eines weiteren Teils des gespülten Katalysators zu einer Regenerierungszone, die ein Wirbelbett umfaßt, und oxidierendes Regenerieren des gespülten Katalysators in der Regenerierungszone, wodurch ein Katalysator erzeugt wird, der im wesentlichen frei von abgelagertem Koks ist; und
(h) Rezirkulieren des regenerierten Katalysators zum Steigrohrreaktor.

2. Verfahren nach Anspruch 1, wobei der Abfluß des Steigrohrs zu einem Abscheider geleitet wird, um Alkylatprodukt und Isobutan zu entfernen, wobei das Isobutan zum Steigrohrreaktor rezirkuliert wird.

3. Verfahren nach Anspruch 1, wobei das alkylierte Produkt ein C₅₊-Alkylat ist.

4. Verfahren nach Anspruch 1, wobei die Umsetzung im Steigrohr in Gegenwart von Wasserstoff erfolgt.

5. Verfahren nach Anspruch 1, wobei die Umsetzung im Steigrohr ohne Wasserstoff erfolgt.

6. Verfahren nach Anspruch 4, wobei ein Bereich der Molverhältnisse zwischen Wasserstoff und Olefin von 0,1:1 bis 1000:1 angewendet wird.

7. Verfahren nach Anspruch 4, wobei ein Bereich der Molverhältnisse zwischen Wasserstoff und Olefin von 2:1 bis 50:1 angewendet wird.

8. Verfahren nach Anspruch 1, wobei der Zeolith mit großen Poren aus der Gruppe ausgewählt ist, die aus Faujasiten, Zeolith Beta, ZSM-3, ZSM-4, ZSM-18, ZSM-20, Mordenit, MCM-22, MCM-36, MCM-49, MCM-56 und Zeolith L besteht.

9. Verfahren nach Anspruch 8, wobei die Faujasite aus der Gruppe ausgewählt sind, die aus Zeolith X, Zeolith Y und USY besteht.

10. Verfahren nach Anspruch 1, wobei der Zeolith mit großen Poren teilweise oder vollständig mit einem Kation oder Kationen, ausgewählt aus Metallen der Seltenen Erden, ausgetauscht ist.

11. Verfahren nach Anspruch 10, wobei der Zeolith mit großen Poren REY oder REUSY ist.

12. Verfahren nach Anspruch 1, wobei der Zeolith mit großen Poren nicht mehr als 1,0 Gew.-% Natrium umfaßt.

13. Verfahren nach Anspruch 1, wobei der den Zeolith mit großen Poren umfassende Katalysator außerdem ein Bindemittel umfaßt.

14. Verfahren nach Anspruch 1, wobei die Alkylierungsbedingungen im Steigrohrreaktor einen Druck im Bereich von 101 bis 791 kPa (0 bis 100 psig) und eine Temperatur unter 483°C einschließen.

15. Verfahren nach Anspruch 1, wobei der Katalysator Partikel umfaßt, deren Größe in einem Bereich von 20 bis 200 µm liegt.

## Revendications

1. Procédé d'alkylation d'isoparaffine-oléfine qui comprend les étapes suivantes, consistant à :
(a) introduire des réactifs d'alimentation frais comprenant des isoparaffines et des oléfines dans un réacteur à colonne montante à lit fluidisé et introduire un catalyseur particulaire fluidisable comprenant une zéolite à larges pores à l'intérieur du réacteur à colonne montante, la zéolite étant capable d'absorber du 2,2,4-triméthylpentane, et les particules de catalyseur ayant une granulométrie dans la gamme de 20 à 200 microns;
(b) faire réagir les réactifs isoparaffines et oléfines dans des conditions d'alkylation, en présence du catalyseur à l'intérieur du réacteur à colonne montante, pour former un effluent de colonne montante comprenant un produit alkylate et de l'isobutane n'ayant pas réagi, les réactifs isoparaffines et oléfines étant en phase vapeur dans le réacteur à colonne montante;
(c) séparer le catalyseur de l'effluent de la colonne montante dans un cyclone;
(d) remettre en circulation au moins une partie de l'effluent de la colonne montante vers le réacteur à colonne montante;
(e) envoyer le catalyseur dans une zone de dépouillement comprenant un lit fluidisé et dépouiller le catalyseur avec un courant à l'intérieur de la zone de dépouillement pour éliminer les hydrocarbures;
(f) envoyer le catalyseur dépouillé de la zone de dépouillement vers une zone de purge comprenant un lit fluidisé et mettre en contact le catalyseur dépouillé avec un gaz pour éliminer l'eau et les hydrocarbures;
(g) remettre en circulation au moins une partie du catalyseur purgé vers le réacteur à colonne montante et envoyer une autre partie du catalyseur purgé vers une zone de régénération comprenant un lit fluidisé, et régénérer par oxydation le catalyseur purgé à l'intérieur de la zone de régénération pour produire un catalyseur essentiellement exempt de dépôt de coke; et
(h) remettre en circulation le catalyseur régénéré vers le réacteur à colonne montante.

2. Procédé selon la revendication 1, dans lequel l'effluent de la colonne montante est envoyé vers un séparateur pour éliminer le produit alkylate et l'isobutane, l'isobutane étant recyclé vers le réacteur à colonne montante.

3. Procédé selon la revendication 1, dans lequel le produit alkylé est un alkylate en C₅+.

4. Procédé selon la revendication 1, dans lequel la réaction à l'intérieur de la colonne montante se fait en présence d'hydrogène.

5. Procédé selon la revendication 1, dans lequel la réaction à l'intérieur de la colonne montante se fait en l'absence d'hydrogène.

6. Procédé selon la revendication 4, dans lequel on emploie une gamme de rapports molaires hydrogène/oléfine de 0,1:1 à 1 000:1.

7. Procédé selon la revendication 4, dans lequel on emploie une gamme de rapports molaires hydrogène/oléfine de 2:1 à 50:1.

8. Procédé selon la revendication 1, dans lequel la zéolite à gros pores est choisie dans le groupe constitué par les faujasites, la zéolite bêta, ZSM-3, ZSM-4, ZSM-18, ZSM-20, la mordénite, MCM-22, MCM-36, MCM-49, MCM-56 et la zéolite L.

9. Procédé selon la revendication 8, dans lequel les faujasites sont choisies dans le groupe constitué par la zéolite X, la zéolite Y et USY.

10. Procédé selon la revendication 1, dans lequel la zéolite à gros pores est échangée partiellement ou totalement avec un cation ou des cations choisis parmi les métaux de terres rares.

11. Procédé selon la revendication 10, dans lequel la zéolite à gros pores est REY ou REUSY.

12. Procédé selon la revendication 1, dans lequel la zéolite à gros pores ne comprend pas plus de 1,0% en poids de sodium.

13. Procédé selon la revendication 1, dans lequel le catalyseur comprenant la zéolite à gros pores comprend en outre un liant.

14. Procédé selon la revendication 1, dans lequel les conditions d'alkylation dans le réacteur à colonne montante comprennent des pressions dans la gamme de 101 à 791 kPa (0 à 100 psig) et des températures inférieures à 483°C.

15. Procédé selon la revendication 1, dans lequel le catalyseur comprend des particules dont la granulométrie s'échelonne de 20 à 200 microns.
